# EUROPÄISCHE PATENTANMELDUNG

(11) **EP 3 270 177 A1**
(43) Veröffentlichungstag der Anmeldung: **17.01.2018**
(21) Anmeldenummer: 17174212.5
(22) Anmeldetag: 02.06.2017
(51) Int. Cl.: G01R 33/36, G01R 19/252

(54) **VERFAHREN ZUM BETREIBEN EINER LOKALSPULE UND LOKALSPULE SOWIE MAGNETRESONANZTOMOGRAPH**

(71) Anmelder: Siemens Healthcare GmbH, 91052 Erlangen (DE)
(72) Erfinder: Fackelmeier, Andreas, 91177 Thalmässing (DE)

(57) **Zusammenfassung**

Die Erfindung betrifft Verfahren zum Betreiben einer Lokalspule (6) für einen Magnetresonanztomographen (2) mit , welche eine Empfangsantenne (16) und einen Signalumsetzer (18) aufweist und signaltechnisch mittels einer Signalleitung (8) mit einer Patientenliege (4) gekoppelt ist, wobei die Empfangsantenne (16) ein analoges Magnetresonanzsignal (22) in einem ersten Signalfrequenzbereich (24) empfängt, wobei das analoge Magnetresonanzsignal (22) mittels des Signalumsetzers (18) in ein digitales Magnetresonanzsignal (34) gewandelt und derart frequenzverschoben wird, dass das digitale Magnetresonanzsignal (34) in einen mit dem ersten Signalfrequenzbereich (24) nicht überlappenden, vorzugsweise gegenüber diesem höherfrequenten, zweiten Signalfrequenzbereich (38) versetzt wird.

## Beschreibung

Die Erfindung betrifft ein Verfahren zum Betreiben einer Lokalspule für einen Magnetresonanztomographen , welche eine Empfangsantenne und einen Signalumsetzer aufweist und signaltechnisch mittels einer Signalleitung mit einer Patientenliege gekoppelt ist, wobei die Empfangsantenne ein analoges Magnetresonanzsignal in einem ersten Signalfrequenzbereich empfängt. Die Erfindung betrifft weiterhin eine Lokalspule sowie einen mit einer solchen Lokalspule ausgestatteten Magnetresonanztomograph.

Ein bildgebender Magnetresonanztomograph umfasst typischerweise eine Patientenliege für einen zu untersuchenden Patienten, welche in einem zylindrischen Messraum positioniert beziehungsweise positionierbar ist. Im Messraum wird im Betrieb beziehungsweise während einer Magnetresonanzuntersuchung mittels einer sogenannten Ganzkörperspule (body coil) ein starkes (statisches) Magnetfeld erzeugt, wobei zusätzlich in der Regel Gradientenspulen vorgesehen sind, mittels welcher ein Magnetfeldgradient zur Verbesserung der räumlichen Auflösung erzeugbar ist. Im Betrieb werden durch ein magnetisches Wechselfeld des Magnetresonanztomographen Kernspins von Atomen im Körper des Patienten an einer Magnetresonanzfrequenz beziehungsweise Larmorfrequenz angeregt. Unter Larmorfrequenz ist hierbei insbesondere die (Präzessions-)Frequenz der Kernspins im (statischen) Magnetfeld des Magnetresonanztomographens zu verstehen. Die resultierenden Magnetresonanzsignale unterschiedlicher Materialien weisen unterschiedliche Relaxationsverhalten auf, sodass aufgrund des Relaxationsverhaltens ein Rückschluss auf das Innere des Körpers des Patienten getroffen werden kann.

Bei einer solchen Magnetresonanzuntersuchung werden zur Signalerfassung dieser Magnetresonanzsignale beziehungsweise Bilddaten in der Regel so genannte Lokalspulen eingesetzt, welche in der Nähe des zu untersuchenden Körperbereichs des Patienten positioniert werden.

Die Lokalspulen weisen typischerweise Empfangsantennen-Baugruppen auf, welche zumindest eine, häufig aber mehrere Empfangsantennen, meist in Form von Leiterschleifen, aufweisen. Die empfangenen analogen Magnetresonanzsignale werden in der Regel noch in der Lokalspule mittels eines Signalumsetzers vorverstärkt und aus dem zentralen Bereich der Magnetresonanzanlage über Signalleitungen ausgeleitet und einem geschirmten Empfänger einer Signalverarbeitungseinrichtung des Magnetresonanztomographen zugeführt.

Die Übertragung der Magnetresonanzsignale beziehungsweise der Bilddaten erfolgt hierbei in der Regel von der Lokalspule zu der Patientenliege und von dieser zur Signalverarbeitungseinrichtung. Die elektrischen oder elektronischen Komponenten der Lokalspule sind hierbei vorzugsweise möglichst energiesparend ausgeführt, sodass keine unerwünschten Wärmeverluste erzeugt werden. Des Weiteren sind die Komponenten möglichst nichtmagnetisch, sodass sie die zu empfangenden Magnetresonanzsignale nicht beeinflussen. Weiterhin sollten die übertragenen Magnetresonanzsignale im Signalfrequenzbereich der empfangenen Magnetresonanzsignale möglichst keine Störsignale erzeugen.

Zur Signalübertragung der empfangenen Magnetresonanzsignale ist es beispielsweise denkbar, dass die analogen Magnetresonanzsignale der Empfangsantenne in digitale Magnetresonanzsignale gewandelt werden. Lokalspulen, bei welchen die Magnetresonanzsignale digitalisiert werden, werden als sogenannte digitale Lokalspulen bezeichnet. Derartige digitale Lokalspulen weisen beispielsweise FPGAs (Field Programmable Gate Arrays) oder Signal-Buffer-Bausteine zur Erzeugung der digitalen Magnetresonanzsignale auf.

Eine Übertragung der digitalisierten Magnetresonanzsignale von der Lokalspule zur Patientenliege wäre in einem Basisband technisch vergleichsweise einfach realisierbar und weist weiterhin einen reduzierten Energieverbrauch der beteiligten elektronischen Komponenten auf.

Nachteiligerweise treten jedoch bei einer solchen Digitalisierung leistungsstarke Signalanteile über einen großen Signalfrequenzbereich von wenigen Hz (Hertz) bis in den GHz-Bereich auf. Dadurch überlappt der Signalfrequenzbereich der digitalisierten Magnetresonanzsignale mit dem Signalfrequenzbereich der zu empfangenden (analogen) Magnetresonanzsignale. Folglich sind die digitalisierten Magnetresonanzsignale typischerweise starke Störsignale für die zu empfangenden Magnetresonanzsignale der Empfangsantennen, welche häufig nicht ausreichend mittels Schirmungen der Signalleitungen gedämpft werden können. Problematisch ist weiterhin, dass in dem Signalfrequenzbereich der digitalisierten Magnetresonanzsignale vergleichsweise aufwändige und kostenintensive Mantelwellensperren für die Signalleitungen benötigt werden.

Der Erfindung liegt die Aufgabe zugrunde, ein besonders geeignetes Verfahren zum Betreiben eines Magnetresonanztomographen anzugeben. Insbesondere soll eine zuverlässige Signalübertragung digitalisierter Magnetresonanzsignale von einer Lokalspule zu einer Patientenliege ermöglicht werden, welche den Empfang von analogen Magnetresonanzsignalen nicht nachteilig beeinflusst. Der Erfindung liegt weiterhin die Aufgabe zugrunde, eine besonders geeignete Lokalspule sowie einen mit einer solchen Lokalspule versehenen Magnetresonanztomographen anzugeben.

Hinsichtlich des Verfahrens wird die Aufgabe mit den Merkmalen des Anspruchs 1 und hinsichtlich der Lokalspule mit den Merkmalen des Anspruchs 3 sowie hinsichtlich des Magnetresonanztomographen mit den Merkmalen des Anspruchs 9 erfindungsgemäß gelöst. Vorteilhafte Ausgestaltungen und Weiterbildungen sind Gegenstand der jeweiligen Unteransprüche.

Das erfindungsgemäße Verfahren ist zum Betreiben eines insbesondere bildgebenden Magnetresonanztomographen geeignet und ausgestaltet. Der Magnetresonanztomograph weist hierbei mindestens eine Lokalspule auf, welche mittels einer Signalleitung signaltechnisch mit einer Patientenliege gekoppelt ist. Die insbesondere digitale Lokalspule weist weiterhin eine Empfangsantenne und einen Signalumsetzer auf.

Im Betrieb des Magnetresonanztomographen, das bedeutet während einer Magnetresonanzuntersuchung, werden analoge Magnetresonanzsignale von der Empfangsantenne empfangen. Die analogen Magnetresonanzsignale weisen hierbei einen ersten Signalfrequenzbereich auf, welcher im Wesentlichen dem Frequenzbereich der auftretenden Magnetresonanzfrequenzen beziehungsweise Larmorfrequenzen entspricht.

Das analoge Magnetresonanzsignal wird mittels des Signalumsetzers in ein digitalisiertes beziehungsweise digitales Magnetresonanzsignal gewandelt und derart frequenzverschoben, dass das digitale Magnetresonanzsignal in einen mit dem ersten Signalfrequenzbereich nicht überlappenden, vorzugsweise gegenüber diesem höherfrequenten, zweiten Signalfrequenzbereich versetzt wird. Mit anderen Worten wird das digitale Magnetresonanzsignal im Vergleich zu dem analogen Magnetresonanzsignal bezüglich der Signalfrequenz hochgesetzt, wodurch das digitale Magnetresonanzsignal bei einer Übertragung über die Signalleitung keine Störsignale im Frequenzbereich der zu empfangenden analogen Magnetresonanzsignale erzeugt. Der zweite Signalfrequenzbereich weist hierbei einen gewissen Mindestfrequenzabstand zum ersten Signalfrequenzbereich auf, sodass zuverlässig sichergestellt ist, dass bei einer Übertragung der digitalen Magnetresonanzsignale keine Störungen im ersten Signalfrequenzbereich erzeugt werden. Dadurch ist ein besonders geeignetes Verfahren zum Betreiben des Magnetresonanztomographen realisiert.

Beispielsweise treten bei einem Magnetresonanztomographen mit einer Magnetfeldstärke von 1,5 T (Tesla) die Magnetresonanzfrequenzen (für Wasserstoff-Kernspins) bei etwa 63,6 MHz (Megahertz), bei einem Magnetresonanztomographen mit einer Magnetfeldstärke von 3 T bei etwa 123,2 MHz, und bei einem Magnetresonanztomographen mit einer Magnetfeldstärke von 7 T bei etwa 297,8 MHz auf. Die Bandbreite der auftretenden Magnetresonanzfrequenzen, also der Magnetresonanzsignale, beträgt geeigneterweise wenige Megahertz. Bevorzugterweise erstreckt sich der erste Signalfrequenzbereich hierbei von 0 bis 600 MHz, insbesondere von 0 bis 300 MHz.

In einer vorteilhaften Ausführung werden zur Frequenzverschiebung des digitalen Magnetresonanzsignals eine Leitungscodierung und/oder eine Signalmodulation verwendet. Unter einer Leitungscodierung ist insbesondere eine Zuordnung einer bestimmten (Signal-)Pegelfolge zu einer Bitfolge oder Bitsequenz im Datenstrom (Bitstrom) zu verstehen. Dadurch ist eine besonders geeignete Signalübertragung gewährleistet. Insbesondere wird das digitale Magnetresonanzsignal nicht durch Mischen frequenzverschoben, sondern durch eine Leitungscodierung und/oder Signalmodulation. Dadurch werden keine Lokaloszillatoren benötigt, wodurch der Aufbau des Signalumsetzers beziehungsweise der Lokalspule vereinfacht wird.

In einer denkbaren Ausführungsform werden die empfangenen Magnetresonanzsignale der Empfangsantenne heruntergemischt. Diese analogen Signale werden anschließend digitalisiert. Das resultierende Frequenzspektrum weist hierbei einen hohen Signalpegel bei 0 Hz auf. Durch die Leitungscodierung und/oder Signalmodulation wird dieser Signalpegel zu höheren Frequenzen verschoben. Durch eine solche Leitungscodierung ist das zu übertragende digitale Magnetresonanzsignal durch gezielte Verformung des Signalspektrums beziehungsweise Verschiebung des Signalfrequenzbereichs an die Signalleitung und die auftretenden Magnetresonanzfrequenzen beziehungsweise Larmorfrequenzen anpassbar.

Geeignete Codes zur Leitungscodierung sind zum Beispiel ternäre Codes wie bipolare Return-to-Zero-Codes (RZ-Code) wie RZ-AMI (Return to Zero - Alternate Mark Inversion). Bei einer solchen Leitungscodierung wird ein zu übermittelnder (Daten-)Bitstrom zur Datenübertragung mittels drei unterschiedlichen (Signal-)Pegelwerten codiert, welche als "+1", "0" und "-1" bezeichnet sind. Die Zustände logisch-0 des Bitstroms werden hierbei mit dem Pegel "0" übertragen, wobei die Zustände logisch-1 des Bitstroms alternierend mit den Pegeln "+1" und "-1" übertragen werden.

Denkbar ist weiterhin, dass ein Code für eine einfache und sichere Taktrückgewinnung verwendet wird, wie beispielsweise ein High-Density-Bipolar-Code (HDBn-Code), insbesondere ein HDB3-Code (High-Density-Bipolar-Code dritter Ordnung). HDBn-Code basiert auf einem AMI-Code, bei welchem allerdings lange Nullfolgen zum Synchronisationsverlust führen können. Zur Vermeidung derartiger Synchronitätsverluste werden beim HDBn-Code sogenannte Codeverletzungsregeln genutzt. Bei einem HDB3-Code So wird beispielsweise die vierte Null ("0") in Folge durch eine Eins ("+1", "-1") in falscher Polarität ersetzt.

Weitere mögliche Codes sind beispielsweise Bipolar With 8 Zeros Substitution (B8Z2) Code, Miller-Code oder Manchester-Code, sowie andere Biphasen-Codes. Der B8Z2-Code verhindert einen Synchronisationsverlust bei einem langen Bitstrom von Nullen. Bei einem Miller-Code (Delay-Code) beziehungsweise bei einer digitalen Frequenzmodulation erfolgt nach jedem Nutzdatenbit (logisch-1, logisch-0) ein Signalwechsel zwischen zwei Pegelwerten. Bei einem logisch-1 Bit erfolgt zusätzlich noch ein Signalwechsel in der Bitmitte. Bei dem Manchester-Code wird das Datensignal bei der Kodierung mit einem Taktsignal versehen. Hierzu moduliert eine Bitfolge binär die Phasenlage des Taktsignals. Mit anderen Worten tragen die Flanken des kodierten Signals, bezogen auf das Taktsignal, die Information.

Ebenso sind Non-Return-to-Zero-Codes (NRZ-Code) mit einem Scrambling des Datensignals, also mit einem Austauschen von Bitfolgen bei der Datenübertragung durch solche, die besser an die Eigenschaften des Übertragungskanals der Signalleitung angepasst sind, denkbar.

Zur Verbesserung der Datenrate ist es weiterhin möglich, dass über eine ternäre Codierung hinaus auch eine mehrstufige Puls Amplituden Modulation (PAM) verwendet wird. Dadurch ist eine besonders effektive und effiziente Signalmodulation bezüglich der spektralen Leistungsverteilung der digitalen Magnetresonanzsignale realisiert.

Die erfindungsgemäße (digitale) Lokalspule ist für einen Magnetresonanztomographen geeignet und eingerichtet. Die Lokalspule weist mindestens eine Empfangsantenne auf, welche an einen Signalumsetzer angeschlossen ist. An den Signalumsetzer ist eine Signalleitung geführt, mittels welcher die Lokalspule an eine Patientenliege des Magnetresonanztomographen signaltechnisch gekoppelt oder koppelbar ist.

Die Lokalspule ist im Montagezustand zur Durchführung des vorstehend beschriebenen erfindungsgemäßen Verfahrens geeignet und eingerichtet. Zu diesem Zwecke weist die Lokalspule beispielsweise einen in den Signalumsetzer integrierten Controller (Steuergerät) auf, welcher hierbei allgemein - programm- und/oder schaltungstechnisch - zur Durchführung des vorstehend beschriebenen Verfahrens eingerichtet ist. Der Controller ist somit konkret dazu eingerichtet die analogen Magnetresonanzsignale mit dem ersten Signalfrequenzbereich zu digitalisieren und insbesondere mittels einer Leitungscodierung und/oder Signalmodulation als digitale Magnetresonanzsignale mit dem zweiten Signalfrequenzbereich in die Signalleitung einzuspeisen.

In einer denkbaren Ausführungsform ist der Controller zumindest im Kern durch jeweils einen Mikrocontroller mit einem Prozessor und einem Datenspeicher gebildet, in dem die Funktionalität zur Durchführung des erfindungsgemäßen Verfahrens in Form einer Betriebssoftware (Firmware) programmtechnisch implementiert ist, so dass das Verfahren - gegebenenfalls in Interaktion mit einem Röntgengerätbenutzer - bei Ausführung der Betriebssoftware in dem Mikrocontroller automatisch durchgeführt wird.

Der Controller kann im Rahmen der Erfindung alternativ aber auch durch nicht-programmierbare Bauteile, zum Beispiel anwendungsspezifischen integrierten Schaltkreise (ASICs), gebildet sein, in denen die Funktionalität zur Durchführung des erfindungsgemäßen Verfahrens mit schaltungstechnischen Mitteln implementiert ist.

In einer bevorzugten Ausbildung weist der Signalumsetzer zur Frequenzverschiebung der digitalen Magnetresonanzsignale einen Logik- beziehungsweise Digitalbaustein auf. Mit anderen Worten ist der Controller insbesondere als Logikbaustein ausgeführt, wobei der Code zur Leitungscodierung vorzugsweise an den Logikbaustein angepasst ist, sodass dieser besonders einfach umgesetzt werden kann. So ist beispielsweise eine Manchestercodierung einfach mittels einer EXOR-Verknüpfung des Taktes mit dem Bitstrom realisierbar. Dies bedeutet, dass das Hochsetzen der Signalfrequenzen auf einem Codieren des Basisbandbitstroms des digitalen Magnetresonanzsignals mit Hilfe des Logikbausteins basiert. Insbesondere erfolgt somit kein hochmischen der Magnetresonanzsignale in den zweiten Signalfrequenzbereich mittels Mischer-Bausteinen.

Im Gegensatz zum Stand der Technik, bei welchem FPGAs (Field Programmable Gate Arrays) oder Signal-Buffer-Bausteine verwendet werden, wird bei der erfindungsgemäßen digitalen Lokalspule lediglich ein Logik- oder Gatterbaustein, beispielsweise ein IC mit sechs Pins, benötigt. Dadurch weist der Signalumsetzer einen besonders niedrigen Energieverbrauch und einen besonders geringen Bauraumbedarf auf. Des Weiteren werden störende Einflüsse durch die empfangenen Magnetresonanzsignale vermieden und Probleme durch magnetische Materialien in den Komponenten der Lokalspule vorteilhaft reduziert. Dadurch ist eine besonders geeignete Lokalspule für einen Magnetresonanztomographen ermöglicht.

In einer geeigneten Weiterbildung ist zwischen dem Signalumsetzer und der Signalleitung ein Hochpassfilter zur Dämpfung von Signalfrequenzen des digitalisierten Magnetresonanzsignals außerhalb des zweiten Signalfrequenzbereichs angeordnet. Der Hochpassfilter ist beispielsweise ausgangsseitig an dem Signalumsetzers angeordnet. Nach dem Leitungscodieren ist es möglich, dass das digitalisierte Magnetresonanzsignal noch Signalanteile in einem Frequenzbereich zwischen 0 Hz und der Magnetresonanzfrequenz aufweist, und somit bei einer Übertragung den Empfang der Empfangsantenne stören kann.

Durch das Herausfiltern dieser störenden Signalanteile wird das digitale codierte Magnetresonanzsignal lediglich geringfügig geändert, ohne dabei jedoch die Übertragung über die Signalleitung nachteilig zu beeinflussen. Der Hochpassfilter ist somit insbesondere dazu geeignet und eingerichtet Signale oder Signalanteile in dem zweiten Signalfrequenzbereich durchzulassen und Signale oder Signalanteile in dem ersten Signalfrequenzbereich zu dämpfen. Mit anderen Worten entspricht der Transmissionsbereich des Hochpassfilters im Wesentlichen dem zweiten Signalfrequenzbereich. Dadurch ist eine besonders effektive und zuverlässige Signalübertragung ermöglicht.

In einer zweckmäßigen Ausgestaltung ist die Signalleitung als eine symmetrische Zweidrahtleitung mit einer Anzahl von Mantelwellensperren ausgeführt, welche eine symmetrische Hochpassleitung bilden. Dadurch wird die Zuverlässigkeit der Signalübertragung weiter verbessert. Das erfindungsgemäße Verfahren überträgt sich hierbei vorteilhaft auf einen vereinfachten Aufbau der Mantelwellensperren, wodurch die Signalleitung einen geringeren Leitungsdurchmesser sowie ein reduziertes Baugewicht aufweist.

In einer möglichen Ausgestaltungsform weist die Signalleitung zusätzlich eine Schirmung auf. Die Mantelwellensperren weisen geeigneterweise diskrete oder mit verteilten Bauteilen aufgebaute Hochpassfilter höherer Ordnung mit Kondensatoren und Spulen auf Platinen beziehungsweise Leiterplatten auf. Die Mantelwellensperren sind hierbei in bestimmten Abständen zueinander zur Bildung der Hochpassleitung in die Signalleitung eingebracht. Im Transmissionsbereich dieser Hochpassleitung werden die digitalen Magnetresonanzsignale von der Lokalspule zu dem Patiententisch übertragen, wobei auf der zweiten Leitung Steuer- und Taktsignale in entgegengesetzter Richtung von dem Patiententisch zu der Lokalspule geführt werden.

In einer denkbaren Ausgestaltungsform erfolgt eine Energieübertragung zur Lokalspule kombiniert mit der Signalleitung oder mittels einer separaten Leitung, bei welcher an der Position der Mantelwellensperren ein Tiefpass oder eine Bandsperre eingefügt ist.

In einer vorteilhaften Ausführung weist die Signalleitung einen Signalanschluss oder Signalstecker auf, welcher zumindest teilweise für eine drahtlose Signalübertragung an die Patientenliege ausgebildet ist. In einer geeigneten Ausführung ist der Signalanschluss insbesondere als ein kontaktloser NFC-Steckverbinder (Near Field Communication, Nahfeldkommunikation) ausgebildet. Dadurch ist eine besonders zweckmäßige signaltechnische Kopplung zwischen der Lokalspule und dem Patiententisch realisiert, welche insbesondere einer Reduzierung der Kontaktzahl bei der Steckverbindung zuträglich ist.

In einer bevorzugten Anwendung wird die Lokalspule bei einem Magnetresonanztomographen eingesetzt und mittels der Signalleitung an die Patientenliege signaltechnisch gekoppelt beziehungsweise angeschlossen. Durch die erfindungsgemäße Lokalspule ist ein besonders geeigneter und energiesparender Magnetresonanztomograph realisiert.

Nachfolgend ist ein Ausführungsbeispiel der Erfindung anhand einer Zeichnung näher erläutert. Darin zeigen in schematischen und vereinfachten Darstellungen:
- FIG 1: ausschnittsweise ein Magnetresonanztomograph mit einer Patientenliege und mit einer Lokalspule,
- FIG 2: die Lokalspule mit einer Empfangsantenne und mit einem Signalumsetzer sowie mit einem Signalkabel, und
- FIG 3: ein Kodierungsdiagramm eines Datensignals mittels eines Manchester-Codes.

Einander entsprechende Teile und Größen sind in allen Figuren stets mit den gleichen Bezugszeichen versehen.

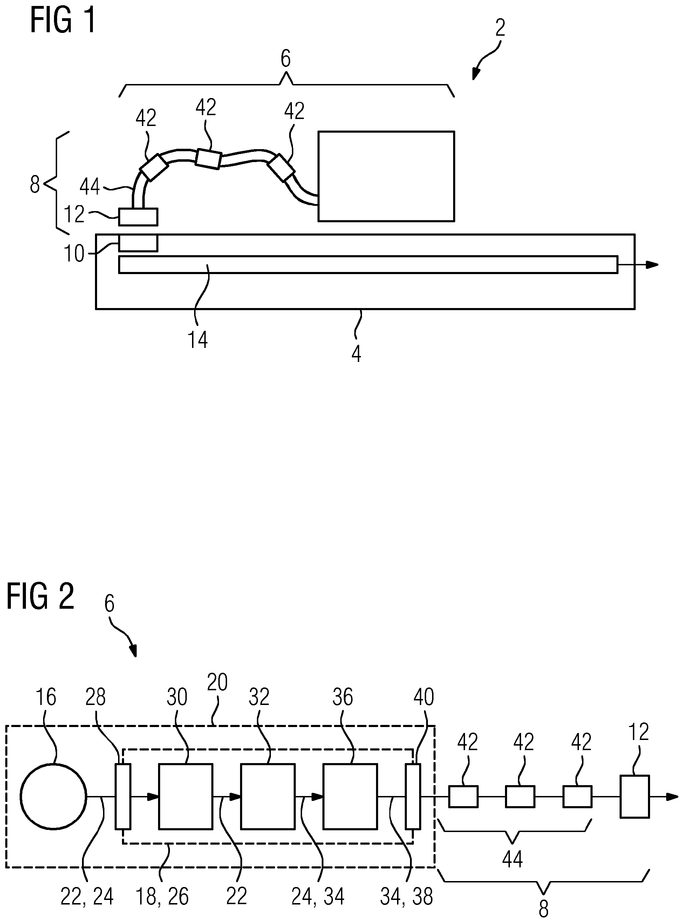

In der FIG 1 ist ausschnittsweise ein bildgebender Magnetresonanztomograph 2 mit einer Patientenliege 4 und einer digitalen Lokalspule 6 dargestellt. Die Lokalspule 6 ist hierbei mittels einer Signalleitung 8 signaltechnisch an die Patientenliege 4 gekoppelt.

Die Patientenliege 4 weist einen Leitungsanschluss 10 für einen Signalanschluss 12 der Signalleitung 8 auf, welcher an eine integrierte Datenleitung 14 geführt ist. Der Signalanschluss 12 ist beispielsweise als ein draht- beziehungsweise kontaktloser NFC-Steckverbinder ausgeführt. Die Datenleitung 14 ist ausgangsseitig, beispielsweise mittels eines Liegenfusssteckers, an eine nicht näher dargestellte Signalverarbeitungseinrichtung geführt, mittels welcher die im Betrieb von der Lokalspule 6 empfangenen Bilddaten ausgewertet und optisch dargestellt werden.

Die in FIG 2 einzeln dargestellte Lokalspule 6 weist mindestens eine Empfangsantenne 16 und einen an die Signalleitung 8 geführten Signalumsetzer 18 auf. Die Empfangsantenne 16 und der Signalumsetzer 18 sind beispielsweise in einer Matte oder einem Gehäuse 20 angeordnet, aus welchem die Signalleitung 8 herausragt.

Im Betrieb, das bedeutet während einer Magnetresonanzuntersuchung, wird die Matte 20 der Lokalspule 6 nahe der zu untersuchenden Region beziehungsweise nahe dem zu untersuchenden Volumen positioniert, sodass die Empfangsantenne 16 einen möglichst geringen Abstand hierzu aufweist. Die Empfangsantenne 16 empfängt analoge Magnetresonanzsignale 22 in einem ersten Signalfrequenzbereich 24 aus dieser Region als Bilddaten und führt diese an den Signalumsetzer 18. Der Signalfrequenzbereich 24 erstreckt sich hierbei in einem kleinen, vergleichsweise schmalbandigen, Frequenzbereich um die Magnetresonanzfrequenz des Magnetresonanztomographen 2 herum. Dies bedeutet, dass der Signalfrequenzbereich 24 im Wesentlichen dem Frequenzbereich der auftretenden Magnetresonanzfrequenzen beziehungsweise Larmorfrequenzen entspricht.

Der Signalumsetzer 18 weist ein Hochfrequenz-Gehäuse (Hochfrequenz-Schirmgehäuse) 26 auf. Eingangsseitig ist der Signalumsetzer 18 mit einem Bandpassfilter 28 versehen, dessen Transmissionsbereich an den Signalfrequenzbereich 24 angepasst ist. Der Bandpassfilter 28 ist an einen Multiplexer 30 geführt, mittels welchem - insbesondere im Falle mehrerer Empfangsantennen 16 - mehrere analoge Signalkanäle multiplexbar sind. Das analoge Magnetresonanzsignal 22 wird anschließend einem Analog-Digital-Wandler 32 zugeführt und in ein digitales Magnetresonanzsignal 34 gewandelt.

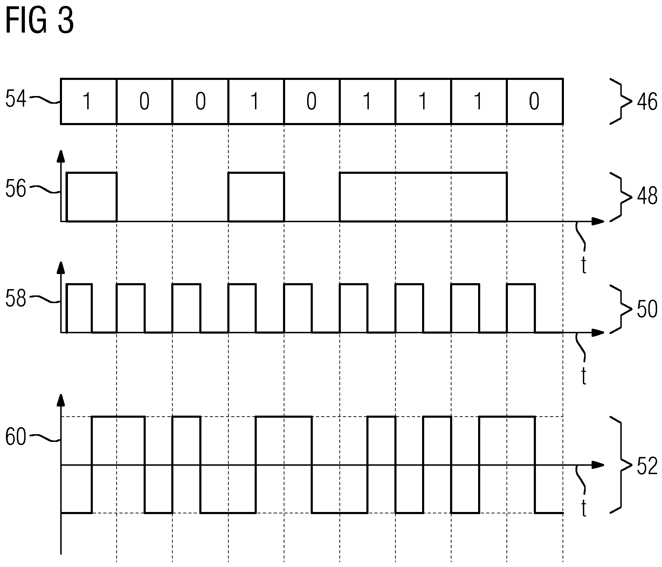

Das digitale Magnetresonanzsignal 34 wird anschließend an einen Controller oder Logikbaustein 36 gesendet, mittels welchem das Magnetresonanzsignal 34 frequenzverschoben wird. Hierzu wird das Magnetresonanzsignal 34 mittels einer Signalbeziehungsweise Leitungscodierung (FIG 3) codiert, und somit dessen Frequenzbereich auf einen zweiten Signalfrequenzbereich 38 hochgesetzt. Der Signalfrequenzbereich 38 des digitalen Magnetresonanzsignals 34 überlappt hierbei nicht mit dem Signalfrequenzbereich 24 des analogen Magnetresonanzsignals 22. Mit anderen Worten wird der Basisbandbitstrom des Magnetresonanzsignals 34 mittels der Codierung des Logikbausteins 36 derart hochgesetzt, dass das Magnetresonanzsignal 34 ohne Störung des Magnetresonanzempfangs der Empfangsantenne 16 mit der Signalleitung 8 an die Patientenliege 4 übertragbar ist.

Um sicherzustellen, dass das übertragene Magnetresonanzsignal 34 keine Signalanteile im ersten Signalresonanzbereich 24 aufweist, wird das Magnetresonanzsignal 34 am Ausgang des Signalumsetzers 18 mittels eines Hochpassfilters 40 in die Signalleitung 8 eingespeist. Das Transmissionsband des Hochpassfilters 40 ist hierbei an den Signalfrequenzbereich 38 des Magnetresonanzsignals 34 angepasst, insbesondere werden Signalfrequenzen aus dem ersten Signalfrequenzbereich 24 gefiltert beziehungsweise gedämpft.

Die Signalleitung 8 ist als eine geschirmte, symmetrische Zweidrahtleitung ausgeführt, wobei in FIG 2 lediglich eine der Drahtleitungen schematisch dargestellt ist. Die Signalleitung 8 weist hierbei eine Anzahl von Mantelwellensperren 42 auf, wobei in den Figuren beispielhaft lediglich jeweils drei Mantelwellensperren 42 dargestellt sind. Die Mantelwellensperren 42 sind hierbei in bestimmten Abständen in die Signalleitung 8 eingesetzt und bilden eine symmetrische Hochpassleitung 44, welche an den Signalanschluss 12 geführt ist.

Der Signalanschluss 12 ist beispielsweise als ein Nahfeldstecker zur zumindest teilweise drahtlosen Signalübertragung an die Patientenliege 4 ausgebildet.

Die FIG 3 zeigt ein Beispiel einer Leitungscodierung mit einem Manchester-Code. Das Diagramm der FIG 3 umfasst vier horizontale, übereinander angeordnete Abschnitte 46 , 48 , 50, 52. Horizontal, das bedeutet auf der X- oder Abszissenachse, ist die Zeit t aufgetragen.

Im oberen Abschnitt 46 der FIG 3 ist ein zu kodierendes Bitsignal oder Bitstrom 54 mit fünf logischen-1 Zuständen und vier logischen-0 Zuständen als ein rechteckiger Kasten dargestellt. Ein entsprechendes Digitalsignal 56, beispielsweise das digitalisierte Magnetresonanzsignal 34 mit dem Signalfrequenzbereich 24, bei dem die logischen-1 Zustände einen hohen Signalpegel und die logischen-0 Zustände einen niedrigen Signalpegel aufweisen ist in dem Abschnitt 48 gezeigt.

In dem Abschnitt 50 ist ein Taktsignal 58 gezeigt, wobei in dem unteren Abschnitt 52 ein anhand des Taktsignals 58 kodiertes Datensignal 60 gezeigt, welches beispielsweise als digitalisiertes Magnetresonanzsignal 34 mit dem Signalfrequenzbereich 38 übertragbar ist.

Bei der gezeigten Leitungscodierung mittels des Manchester-Codes wird die Phasenlage des Taktsignal 58 binär mittels des Datensignals 56 derart moduliert, dass eine fallende Flanke des Datensignals 60 einen logischen-0 Zustand und eine steigende Flanke einen logischen-1 Zustand des Bitstroms 54 beschreibt. Die gezeigte Manchestercodierung ist beispielsweise einfach mittels einer EXOR-Verknüpfung des Logikbausteins 36 durch Verknüpfung des Taktsignals 58 mit dem Bitstrom 54 beziehungsweise Digitalsignals 56 realisiert.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit dem Ausführungsbeispiel beschriebenen Einzelmerkmale auch auf andere Weise miteinander kombinierbar, ohne den Gegenstand der Erfindung zu verlassen.

## Patentansprüche

1. Verfahren zum Betreiben einer Lokalspule (6) für einen Magnetresonanztomographen (2), welche eine Empfangsantenne (16) und einen Signalumsetzer (18) aufweist und signaltechnisch mittels einer Signalleitung (8) mit einer Patientenliege (4) gekoppelt ist,
- wobei die Empfangsantenne (16) ein analoges Magnetresonanzsignal (22) in einem ersten Signalfrequenzbereich (24) empfängt,
- wobei das analoge Magnetresonanzsignal (22) mittels des Signalumsetzers (18) in ein digitales Magnetresonanzsignal (34) gewandelt und derart frequenzverschoben wird, dass das digitale Magnetresonanzsignal (34) in einen mit dem ersten Signalfrequenzbereich (24) nicht überlappenden, vorzugsweise gegenüber diesem höherfrequenten, zweiten Signalfrequenzbereich (38) versetzt wird.

2. Verfahren nach Anspruch 1,
**dadurch gekennzeichnet, dass** zur Frequenzverschiebung des digitalen Magnetresonanzsignals (34) eine Leitungscodierung und/oder eine Signalmodulation verwendet wird.

3. Lokalspule (6) für einen Magnetresonanztomographen (2) zur Durchführung des Verfahrens nach Anspruch 1 oder 2, aufweisend eine Empfangsantenne (16) und einen Signalumsetzer (18) sowie eine Signalleitung (8) zur signaltechnischen Kopplung mit einer Patientenliege (4).

4. Lokalspule (6) nach Anspruch 3,
**dadurch gekennzeichnet, dass** der Signalumsetzer (18) zur Frequenzverschiebung einen Logikbaustein (36) aufweist.

5. Lokalspule (6) nach Anspruch 3 oder 4,
**dadurch gekennzeichnet, dass** zwischen dem Signalumsetzer (18) und der Signalleitung (8) ein Hochpassfilter (40) zur Dämpfung von Signalfrequenzen des digitalisierten Magnetresonanzsignals (34) außerhalb des zweiten Signalfrequenzbereichs (38) angeordnet ist.

6. Lokalspule (6) nach einem der Ansprüche 3 bis 5,
**dadurch gekennzeichnet, dass** die Signalleitung (18) als eine symmetrische Zweidrahtleitung mit einer Anzahl von Mantelwellensperren (42) ausgeführt ist, die eine symmetrische Hochpassleitung (44) bilden.

7. Lokalspule (6) nach einem der Ansprüche 3 bis 6,
**dadurch gekennzeichnet, dass** die Signalleitung (8) einen Signalanschluss (12) aufweist, welcher für eine drahtlose Signalübertragung an die Patientenliege (4) ausgebildet ist.

8. Lokalspule (6) nach Anspruch 7,
**dadurch gekennzeichnet, dass** der Signalanschluss (12) als ein kontaktloser NFC-Steckverbinder ausgeführt ist.

9. Magnetresonanztomograph (2) aufweisend eine Lokalspule (6) nach einem der Ansprüche 3 bis 8 und eine damit signaltechnisch gekoppelte Patientenliege (4).

## Geänderte Patentansprüche

### Geänderte Patentansprüche gemäss Regel 137(2) EPÜ.

1. Verfahren zum Betreiben einer Lokalspule (6) für einen Magnetresonanztomographen (2) , welche eine Empfangsantenne (16) und einen Signalumsetzer (18) aufweist und signaltechnisch mittels einer Signalleitung (8) mit einer Patientenliege (4) gekoppelt ist,
- wobei die Empfangsantenne (16) ein analoges Magnetresonanzsignal (22) in einem ersten Signalfrequenzbereich (24) empfängt, und
- wobei das analoge Magnetresonanzsignal (22) mittels des Signalumsetzers (18) in ein digitales Magnetresonanzsignal (34) gewandelt und derart frequenzverschoben wird, dass das digitale Magnetresonanzsignal (34) in einen mit dem ersten Signalfrequenzbereich (24) nicht überlappenden, gegenüber diesem höherfrequenten, zweiten Signalfrequenzbereich (38) versetzt wird.

2. Verfahren nach Anspruch 1,
**dadurch gekennzeichnet, dass** zur Frequenzverschiebung des digitalen Magnetresonanzsignals (34) eine Leitungscodierung und/oder eine Signalmodulation verwendet wird.

3. Lokalspule (6) für einen Magnetresonanztomographen (2) zur Durchführung des Verfahrens nach Anspruch 1 oder 2, aufweisend eine Empfangsantenne (16) und einen Signalumsetzer (18) sowie eine Signalleitung (8) zur signaltechnischen Kopplung mit einer integrierten Datenleitung (14) einer Patientenliege (4),
- wobei die Empfangsantenne (16) ein analoges Magnetresonanzsignal (22) in einem ersten Signalfrequenzbereich (24) empfängt, und
- wobei der Signalumsetzer (18) das analoge Magnetresonanzsignal (22) in dem ersten Signalfrequenzbereich (24) in ein digitales Magnetresonanzsignal (34) wandelt und derart frequenzverschiebt, dass das digitale Magnetresonanzsignal (34) in einen mit dem ersten Signalfrequenzbereich (24) nicht überlappenden, gegenüber diesem höherfrequenten, zweiten Signalfrequenzbereich (38) versetzt ist.

4. Lokalspule (6) nach Anspruch 3,
**dadurch gekennzeichnet, dass** der Signalumsetzer (18) zur Frequenzverschiebung einen Logikbaustein (36) aufweist.

5. Lokalspule (6) nach Anspruch 3 oder 4,
**dadurch gekennzeichnet, dass** zwischen dem Signalumsetzer (18) und der Signalleitung (8) ein Hochpassfilter (40) zur Dämpfung von Signalfrequenzen des digitalisierten Magnetresonanzsignals (34) außerhalb des zweiten Signalfrequenzbereichs (38) angeordnet ist.

6. Lokalspule (6) nach einem der Ansprüche 3 bis 5,
**dadurch gekennzeichnet, dass** die Signalleitung (18) als eine symmetrische Zweidrahtleitung mit einer Anzahl von Mantelwellensperren (42) ausgeführt ist, die eine symmetrische Hochpassleitung (44) bilden.

7. Lokalspule (6) nach einem der Ansprüche 3 bis 6,
**dadurch gekennzeichnet, dass** die Signalleitung (8) einen Signalanschluss (12) aufweist, welcher für eine drahtlose Signalübertragung an die Patientenliege (4) ausgebildet ist.

8. Lokalspule (6) nach Anspruch 7,
**dadurch gekennzeichnet, dass** der Signalanschluss (12) als ein kontaktloser Near Field Communication-Steckverbinder ausgeführt ist.

9. Magnetresonanztomograph (2) aufweisend eine Lokalspule (6) nach einem der Ansprüche 3 bis 8 und eine damit signaltechnisch gekoppelte Patientenliege (4) mit einer integrierten Datenleitung (14).
